(19) Europäisches Patentamt
European Patent Office
Office européen des brevets

(11) Publication number: **0 430 023 A1**

(12) **EUROPEAN PATENT APPLICATION**

(21) Application number: **90122143.2**

(22) Date of filing: **20.11.90**

(51) Int. Cl.[5]: **C07C 255/41**, C07C 253/30

(30) Priority: **29.11.89 US 442810**

(43) Date of publication of application: **05.06.91 Bulletin 91/23**

(84) Designated Contracting States: **BE CH DE FR GB IT LI NL Bulletin**

(71) Applicant: **BASF Corporation**
**9 Campus Drive**
**Parsippany, NJ 07054(US)**

(72) Inventor: **Goldstein, Steven Ira**
**34 Duncan Drive**
**Latham, New York 12110(US)**
Inventor: **Labrie, Edwin F.**
**R.D. 1**
**Rensselaer, New York 12144(US)**

(74) Representative: **Mutzbauer, Helmut, Dr. et al**
**BASF Aktiengesellschaft Patentabteilung**
**ZSP - C 6 Carl-Bosch-Strasse 38**
**W-6700 Ludwigshafen/Rhein(DE)**

(54) **Process for the preparation of substituted phenylcinnamates.**

(57) This is a process for the preparation of substituted phenylcinnamates, which are in turn used as u.v. absorbers. The process comprises the steps of:

1) charging a diarylketone, cyanoacetic acid ester, catalyst solution, and $C_6$ to $C_8$ hydrocarbon solvent is a reaction vessel with agitation,
2) heating the mixture to reflux, removal of generated water,
3) adding in a stepwise manner the remaining catalyst solution, and
4) recovering the product from the reaction mixture.

EP 0 430 023 A1

## PROCESS FOR THE PREPARATION OF SUBSTITUTED PHENYLCINNAMATES

This invention relates to a process for making ultraviolet radiation absorbing compounds and, more particularly, a novel method for the production of substituted phenyl cinnamates. This novel process provides product in high yields. In the case of liquids, the final products are lighter in color, therefor more desirable. In the case of solid final products the process cycle time has been reduced. This is accomplished while substantially reducing or eliminating the use of aromatic solvents such as benzene, toluene, or xylene. Applicant's process allows for a more efficient and environmentally responsible procedure for producing substituted phenylcinnamates.

Various organic compounds exhibiting the power to absorb ultraviolet radiation are known in the art. Among these known compounds are substituted phenyl cinnamates. These compounds are generally prepared by the condensation of a cyanoacetic acid ester with a diaryl ketone. United States Patent No. 2,623,060 discloses a modified Knoevenagel reaction wherein, diarylketones are reacted with a cyanoacetic acid ester to form the corresponding diaryl cyanoacetic acid ester. This process uses aromatic solvents, the modification being the addition of the solid ammonium acetate in a stepwise manner. Numerous other patents disclose related methods for making diaryl cyanoacetic acid esters and their derivatives. Specific United States patents would include 3,337,357; 3,644,466; 4,178,303; and 4,207,523. These are selected as illustrative examples and are not meant to be construed as a comprehensive list of all patents disclosing this type of reaction. The above patents disclose a general method for making substituted phenyl cinnamates wherein the reactants are combined with an aromatic solvent and catalyst, heated to reflux, water removed, and the product recovered from the solvent. This method generally yields approximately 60 percent to 70 percent product.

What is provided herein is an improved process for preparation of substituted phenyl cinnamates of the formula:

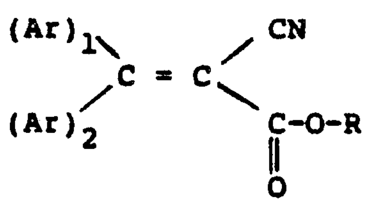

where

$(Ar)_1$ and $(Ar)_2$ are aromatic carboxylic nuclei of the benzene and naphthalene series and are independently selected from phenyl or phenyl substituted with alkyl, halo, alkoxy, carboxy, carbalkoxy, cyano, acetyl, benzyl, phenyl, alkyl phenyl, phenoxyphenyl, alkyl substituted phenoxy, or alkoxy phenyl substituted phenyl, and naphthyl;

and R is an $C_1$-$C_{17}$, linear or branched, unsubstituted or substituted alkyl with halo, cyano, alkyl, alkoxy, alkoxyalkyl, alkoxyalkyleneoxy.

The process for the preparation of the substituted phenyl cinnamates comprises the steps of:

(a) heating to reflux a mixture of a diaryl ketone, an ester of cyanoacetic acid, ammonium acetate, glacial acetic acid, and an aliphatic or cycloaliphatic, $C_6$ to $C_8$, hydrocarbon solvent, and removing water generated,

(b) adding an ammonium acetate-glacial acetic acid solution in a stepwise manner to said heated mixture, while removing the water of formation, and

(c) recovering the resulting substituted phenyl cinnamates acrylic acid ester from the mixture.

In the preferred embodiments of this invention, $(Ar)_1$ and $(Ar)_2$ are phenyl, R is ethyl or ethylhexyl, and the $C_6$ to $C_8$ hydrocarbon solvent is heptane.

In the process of this invention a diaryl ketone, a aliphatic or cycloaliphatic, $C_6$ to $C_8$ hydrocarbon, e.g. hexane, cyclohexane, heptane, capable of solubilizing the starting materials, ammonium acetate-glacial acetic acid solution and a cyanoacetic acid ester are charged under nitrogen to a reaction vessel. In general the ratio of reactants used is about a 1:1 molar ratio of the diarylketone to the ester of cyanoacetic acid. In the preferred embodiment, a slight excess of the ester is used, such as 1:1.05 to about 1:1.5, diaryl ketone:ester. The amount of ammonium acetate present is not critical as long as there is an amount present

sufficient to catalyze the reaction. A typical amount of catalyst present including the initial amount charged and the subsequent amount added would be from about 0.3 moles to about 0.5 moles per mole of diaryl ketone. The reaction mixture is heated to reflux at about 95 °C to 110 °C, most preferably 100 °C to about 104 °C and allowed to continue at reflux for about one hour while collecting the water generated. After the mixture has refluxed for one hour, additional ammonium acetate-glacial acetic acid solution is added at a predetermined rate, while maintaining reflux. U.S. patent 2,623,060 discloses a stepwise addition of ammonium acetate solid to a reaction mixture. The addition of a solid, such as that which is disclosed in '060, to a reaction mixture at reflux presents obvious problems. Either the contents of the reaction vessel must be cooled to allow the reactor to be opened for the catalyst addition or extensive modification must be made to provide for delivery of the solid into the reactor while at elevated temperature. This aspect of Applicant's invention obviates the above problems. Simple, accurate addition of the catalyst solution may be provided through a metering pump.

When the catalyst addition is complete, the reaction is allowed to continue at reflux for one hour. After about one hour, the reaction mixture is subjected to a series of washes. The contents of the reaction vessel are then passed through a filter. The filtrate is allowed to separate and the aqueous phase is drained and discarded. The filtrate is again subject to a water wash and padded with nitrogen. Common salt, sodium chloride, may be added to aid separation of the aqueous and organic phases. When the separation of phases is complete, the aqueous phase is drained. The solvent is removed by vacuum distillation or wipe film evaporation yielding the substituted phenylcinnamate.

In a preferred embodiment of the invention in which an alkyl ester of cyanoacetic acid such as ethyl cyanoacetate is used as the cyanoacetic acid ester and benzophenone is the diaryl ketone, the resulting product is sparingly soluble in the aliphatic hydrocarbon solvent at temperatures less than about 70 °C. The solvent-product mixture is clarified with nitrogen. Then temperature of the product-solvent mixture is slowly reduced over a five and one half hour period to about 25 °C. The product precipitates out of the solvent mixture at this point. Product and solvent mixture are then blown, with filtered nitrogen, through a pressure filter fitted with a cloth. The solvent mixture is recovered and recycled. The product is collected in the filter, purified with aqueous or aqueous-alcohol washes and dried for thirty minutes with nitrogen.

When practicing the invention in its preferred embodiments, i.e., benzophenone as the diaryl ketone, ethylcyanoacetate or ethylhexylcyanoacetate as the cyanoacetic acid derivative, heptane as the non-aromatic hydrocarbon solvent, and ammonium acetate as the catalyst predissolved in glacial acetic acid, the process generates product yields in excess of about 90 percent. Furthermore, the product of this process is generally lighter in color than a comparable process using aromatic solvents, and therefor more desirable.

Solid final product color determinations are made via the APHA color method. The solid product is dissolved in 2-butanone, in a 1:9, product to solvent, weight ratio and the resulting solution compared against a 2-butanone standard, using a Hellige Aqua Tester equipped with a "Color of Water" wheel.

The color of liquid final products is a determination of VCS Color via CIE Colorimetry. The color of a toluene solution of the liquid UV absorber is measured on a visible spectrophotometer, integrated in terms of CIE illuminant C and plotted on a chromaticity diagram on which VCS color standards are also plotted. The VCS color equivalent is estimated to the nearest 0.1 unit.

Products made using applicant's improved process have averaged a VCS color equivalent of 1.7 compared to previous process averages of 2.2.

The following Examples serve to illustrate the present invention and are not meant to be used as an inferred limitation.

Example 1

Preparation of

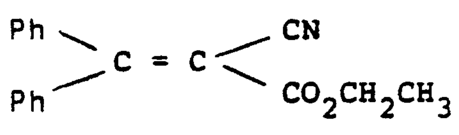

Into a 500 ml flask fitted with a stirrer, thermometer, reflux condenser, watertrap, and heating mantle there are charged the following:

45.5g ethylcyanoacetate (0.4 mole)

61g benzophenone (0.3 mole)
5.8g ammonium acetate
17.3g glacial acetic acid and
130ml heptane

The above charge is stirred for 1 hour at the reflux-temperature. 1.5 ml of ammonium acetate-acetic acid catalyst are added every hour for 18 hours. The reaction mixture is cooled to 25°C. The solid is filtered, washed with water then methanol water and dried to give 72g (91% yield).

Example 2

Preparation of

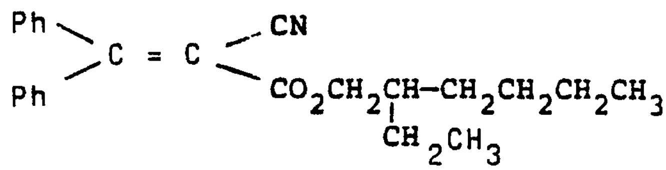

Into a 500 ml flask fitted with a stirrer, thermometer, reflux condenser, water trap and heating mantle are charged the following:

70g 2-ethylhexylcyanoacetate (0.4 mole)
61g benzophenone (0.3 mole)
7.2g ammonium acetate
25.7g glacial acetic acid
112ml cyclohexane

The above charge is stirred for 1 hour at the reflux temperature. 1.5ml of ammonium acetate-acetic acid catalyst are added every hour for 22 hours. The reaction mixture is cooled to 60°C and washed with water. The water separated and solvent and unreacted starting material removed, solvent and unreacted starting material removed by distillation. Purification of product gave 110g (92% yield).

## Claims

**1.** A process for the preparation of substituted phenyl cinnamates comprising the steps of:
(a) heating to reflux a mixture of a diarylketone, an ester of cyanoacetic acid, ammonium acetate-glacial acetic acid, and an aliphatic or cycloaliphatic, $C_6$ to $C_8$ hydrocarbon solvent, and removing water present in said mixture;
(b) adding an ammonium acetate-glacial acetic acid solution in a stepwise manner, to said heated mixture, while removing water of formation; and
(c) recovering the resulting substituted phenyl cinnamate from the hydrocarbon solvent.

**2.** A process according to claim 1 wherein a portion of the mixture of diarylketone, the ester of cyanoacetic acid, and solvent is supplied by recycled, unreacted starting materials and solvent from a previous synthesis.

**3.** A process according to claim 1 or 2 wherein said diarylketone is benzophenone and said ester of cyanoacetic acid is chosen from a class consisting of alkylesters of cyanoacetic acid.

**4.** A process according to claims 1 to 3 wherein said cyanoacetic acid ester is ethyl cyanoacetate.

**5.** A process according to claims 1 to 3 wherein said cyanoacetic acid ester is ethylhexyl cyanoacetate.

**6.** A process according to claims 1 to 5 wherein said solvent is heptane.

**7.** A process according to claims 1 to 6 wherein said mixture is refluxed at about 100°C to about 104°C.

**8.** A process according to claim 3 wherein said resulting substituted phenyl cinnamate is sparingly soluble

in the hydrocarbon solvent at temperatures below about 70°C and is recovered by reducing said temperature to a point at which said substituted phenyl cinnamate is precipitated out of said aliphatic hydrocarbon solvent and recovered from said solvent by filtration.

**9.** A process according to claim 8 wherein unreacted starting products and solvent are recycled.

European Patent Office

# EUROPEAN SEARCH REPORT

Application Number

**EP 90 12 2143**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-1 342 572 (GAF)<br>* Abstract; page 6, column 2; examples 1,10; page 1, column 2, line 2 from bottom - page 2, column 1, line 10 from top *<br>- - - | 1-9 | C 07 C 255/41<br>C 07 C 253/30 |
| D,A | US-A-2 623 060 (E.J. CRAGOE, Jr.)<br>* Complete document *<br>- - - | 1-9 | |
| A | JOURNAL OF ORGANIC CHEMISTRY, vol. 15, no. 2, March 1950, pages 381-390, The Williams & Wilkins Co., Baltimore, US; E.J. CRAGOE, Jr. et al.: "The synthesis of alpha,alpha-disubstituted succinic acids from ethyl alkylidenecyanoacetates"<br>* Pages 381-383 *<br>- - - | 1-9 | |
| A | "Organic Reactions", vol. 15, pages 204,238,239,265,414, John Wiley & Sons, Inc. New York, US; chapter 2, G. JONES: "The Knoevenagel Condensation"<br>* Pages 238,238,265,414 *<br>- - - - - | 1-9 | |

TECHNICAL FIELDS SEARCHED (Int. Cl.5)

C 07 C 253/00
C 07 C 255/00

The present search report has been drawn up for all claims

| Place of search | Date of completion of search | Examiner |
|---|---|---|
| The Hague | 11 March 91 | WRIGHT M.W. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document